# EUROPEAN PATENT APPLICATION

(11) **EP 1 922 978 A1**
(43) Date of publication of application: **21.05.2008**
(21) Application number: 06783231.1
(22) Date of filing: 08.09.2006
(51) Int. Cl.: A61B 1/00, A61B 5/07

(54) **DEVICE TO BE INTRODUCED INTO SUBJECT**

(30) Priority: 09.09.2005 JP 2005262044
(71) Applicant: Olympus Corporation, Tokyo 151-0072 (JP)
(72) Inventor: HONDA, Takemitsu c/o OLYMPUS MEDICAL SYSTEMS CORP.,, Tokyo 151-0072 (JP); HOMAN, Masatoshi c/o OLYMPUS MEDICAL SYSTEMS CORP.,, Tokyo 151-0072 (JP); ORIHARA, Tatsuya c/o OLYMPUS MEDICAL SYSTEMS CORP.,, Tokyo 151-0072 (JP); FUJIMORI, Noriyuki c/o OLYMPUS CORPORATION,, Tokyo 151-0072 (JP); SUZUSHIMA, Hiroshi c/o OLYMPUS CORPORATION,, Tokyo 151-0072 (JP); NAKATSUCHI, Kazutaka c/o OLYMPUS MEDICAL SYSTEMS CORP.,, Tokyo 151-0072 (JP)
(74) Representative: von Hellfeld, Axel
(86) International application number: PCT/JP2006/317871
(87) International publication number: WO 2007/029820

(57) **Abstract**

In a capsule casing 16 as an exterior case that includes end cover casings 16a and 16b and body cover casings 16a and 16b, planar portions 16f and 16g are formed at portions of a circumferential surface of the body cover casings 16c and 16d, and imaging units 14a and 14b are arranged inside the capsule casing 16 at positions at which imaging inside of body cavities of a subject is possible through the planar portions 16f and 16g, thereby preventing a lens effect, eliminating image failures to improve images, and enhancing image quality.

## Description

### TECHNICAL FIELD

The present invention relates to a body-insertable apparatus such as a capsule endoscope which is swallowed into a body of a subject.

### BACKGROUND ART

Recently, in the field of endoscope, a capsule endoscope equipped with an imaging function and a radio communication function has appeared. The capsule endoscope is configured to sequentially take images during an observation period after the capsule endoscope is swallowed from the mouth of an examinee as a subject (human body) for observation (examination) until naturally discharged out of a body of the examinee, while passing through inside of organs (inside of body cavities) such as a stomach and a small intestine according to the peristalsis thereof.

Moreover, image data obtained inside the body cavities by the capsule endoscope during this observation period while moving inside these organs is sequentially transmitted to the outside by the radio communication function such as radio communications, and stored in a memory provided in an external receiving device. If the examinee carries the external device having such radio communication function and the memory function, the examinee can freely move without having any inconvenience even during the observation period from swallowing the capsule endoscope until it is discharged. After the observation, doctors can perform diagnosis based on the image data stored in the memory of the receiving device by displaying images of the body cavities on a display unit such as a display (for example, see Patent Document 1).

Patent Document 1: Japanese Patent Application Laid-open No. 2003-19111

### DISCLOSURE OF INVENTION

### PROBLEM TO BE SOLVED BY THE INVENTION

However, since an outer surface of the capsule endoscope is formed in a cylindrical shape, there has been a problem that an image failure such as image distortion, flare, and shading due to a lens effect of the outer surface, tends to occur if an imaging unit is arranged at an imaging position through this outer surface.

The present invention has been achieved in view of the above problems, and it is an object of the present invention to provide a body-insertable apparatus capable of eliminating the image failures to improve images, and of enhancing image quality.

### MEANS FOR SOLVING PROBLEM

To solve the above problems and to achieve the object of the present invention, a body-insertable apparatus inserted inside a body of a subject to capture an image inside the body of the subject according to the present invention includes an exterior case that has at least a cylindrical circumferential surface and closes an inside thereof in a water tight manner; a planar portion that forms a plane at a portion of the cylindrical circumferential surface; and an imaging unit that is arranged inside the exterior case at a position at which an imaging of an outside is possible through the planar portion.

In the body-insertable apparatus according to the invention as set forth in claim 2, the planar portion is formed so as to dent from the circumferential surface to have a concave cross-section.

In the body-insertable apparatus according to the invention as set forth in claim 3, the planar portion is formed so as to protrude from the circumferential surface to have a convex cross-section.

In the body-insertable apparatus according to the invention as set forth in claim 5, the exterior case is formed with a first and a second case members having an axisymmetric shape with respect to a center axis of a traveling direction of the body-insertable apparatus.

In the body-insertable apparatus according to the invention as set forth in claim 5, the planar portion excluding an imaging region of the imaging unit and the exterior case are formed with a light shielding member.

A body-insertable apparatus inserted into a body of a subject to capture an image inside the body of the subject according to the invention as set forth in claim 6 includes an exterior case that has at least a cylindrical circumferential surface and closes an inside thereof in a water tight manner; a lens unit that is formed to have a curvature at a portion of the cylindrical circumferential surface; and an imaging unit that is arranged inside the exterior case at a position at which an imaging of an outside is possible through the lens unit.

In the body-insertable apparatus according to the invention as set forth in claim 7, the exterior case excluding the lens unit is formed with a light shielding member.

### EFFECT OF THE INVENTION

In the body-insertable apparatus according to the present invention, the planar portion is formed at a portion of the circumferential surface having a cylindrical shape of the exterior case, and the imaging unit is arranged at a position at which imaging of outside is possible through the planar portion. Thus, such effects can be obtained that a lens effect is prevented, image failures are eliminated to improve images, and image quality is enhanced.

### BRIEF DESCRIPTION OF DRAWINGS

FIG. 1 is a system conceptual view showing a concept of a radio in-vivo information acquiring system according to the present invention;
FIG. 2 is a side cross-section showing a schematic configuration of a capsule endoscope according to a first embodiment;
FIG. 3 is an arrow view showing a view taken from a direction of an arrow A shown in FIG. 2;
FIG. 4 is a side cross-section of a capsule casing of that shown in FIG. 2;
FIG. 5 is a schematic block diagram showing an internal circuit configuration of the capsule endoscope;
FIG. 6 is a side cross-section showing a schematic configuration of a capsule endoscope according to a second embodiment; and
FIG. 7 is a side cross-section showing a schematic configuration of a capsule endoscope according to a third embodiment.

### EXPLANATIONS OF LETTERS OR NUMERALS

- 1: SUBJECT
- 2: RECEIVING DEVICE
- 2a: RECEIVING JACKET
- 2b: EXTERNAL DEVICE
- 3: CAPSULE ENDOSCOPE
- 4: DISPLAY DEVICE
- 5: PORTABLE RECORDING MEDIUM
- 11a, 11b: LED
- 12a, 12b: CCD
- 12a1, 12b1: LIGHT RECEIVING SURFACE
- 13a, 13b: IMAGING LENS
- 14a, 14b: IMAGING UNIT
- 15: POWER SOURCE UNIT (BATTERY)
- 16: CAPSULE CASING
- 16a, 16b: END COVER CASING
- 16c, 16d: BODY COVER CASING
- 16e: PARTING LINE
- 16f, 16g: PLANAR PORTION
- 16h, 16i: LENS UNIT
- 17a, 17b: ILLUMINATING/IMAGING BOARD
- 26a, 26b: SIGNAL PROCESSING/CONTROL UNIT
- 27: RADIO UNIT
- 28: RADIO BOARD
- 41a, 41b: LED DRIVING CIRCUIT
- 42a, 42b: CCD DRIVING CIRCUIT
- 43a, 43b: IMAGE PROCESSOR
- 44a, 44b: TIMING GENERATOR AND SYNC GENERATOR
- 45a, 45b: CONTROL UNIT
- 46: TRANSMITTING MODULE
- 47: ANTENNA
- 49: PARAMETER MEMORY
- A1 to An: RECEIVING ANTENNAS

### BEST MODE(S) FOR CARRYING OUT THE INVENTION

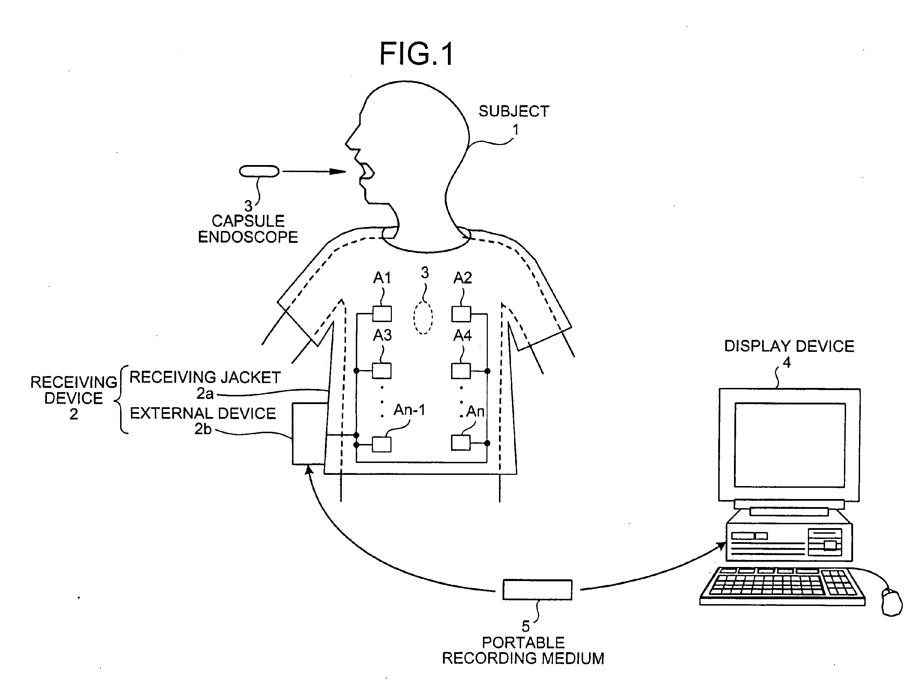

Exemplary embodiments of a body-insertable apparatus according to the present invention will be explained below in detail with reference to the drawings of FIG. 1 to FIG. 7. It should be noted that the present invention is not limited to the embodiments, and the embodiments can be variously modified without departing from the scope of the present invention.

### FIRST EMBODIMENT

FIG. 1 is a system conceptual view showing a concept of a radio in-vivo information acquiring system according to the present invention. In this radio in-vivo information acquiring system, a capsule endoscope is used as an example of a body-insertable apparatus. As shown in FIG. 1, the radio in-vivo information acquiring system includes a swallowable capsule endoscope 3 that is inserted into body cavities of a subject 1 as a radio in-vivo information acquiring system, and a receiving device 2 that is an external device arranged outside the subject 1 and that communicates various types of information with the capsule endoscope 3 by radio communication. Moreover, the radio in-vivo information acquiring system includes a display device 4 that performs image display based on data received by the receiving device 2, and a portable recording medium 5 that performs input and output of data between the receiving device 2 and the display device 4.

The receiving device 2 has a function as a radio receiving unit that receives data of images inside of body cavities that is transmitted from the capsule endoscope 3 by radio communication as shown in FIG. 1. The receiving device 2 includes a receiving jacket 2a that is worn by the subject 1 and that has a plurality of receiving antennas A1 to An, and an external device 2b that performs a signal processing and the like of received radio signals.

The display device 4 is to display the images inside of body cavities acquired by the capsule endoscope 3, and has a configuration similar to a workstation and the like to perform image display based on the data stored in the portable recording medium 5. Specifically, the display device 4 can take a configuration to directly display the images with a CRT display, a liquid crystal display, and the like, or can take a configuration to output the images to another medium such as that of a printer, etc.

The portable recording medium 5 is configured to be connected to the external device 2b and the display device 4, and to enable output and record of information when connected thereto. In the present embodiment, the portable recording medium 5 is inserted into the external device 2b while the capsule endoscope 3 is moving inside the body cavities of the subject 1, and records data that is transmitted from the capsule endoscope 3. After the capsule endoscope 3 is discharged out of the subject 1, in other words, when the imaging of the inside of the subject 1 is completed, the portable recording medium 5 is removed from the external device 2b and inserted into the display device 4 so that the data recorded in the portable recording medium 5 is read out by the display device 4. For example, this portable recording medium 5 is constituted of the CompactFlash (registered trademark) memory and the like, and enables input and output of data between the external device 2b and the display device 4 indirectly through the portable recording medium 5. Unlike the case in which the external device 2b and the display device 4 are linked by wired connection, the subject 1 can freely move even while the imaging of the inside of body cavities is being performed.

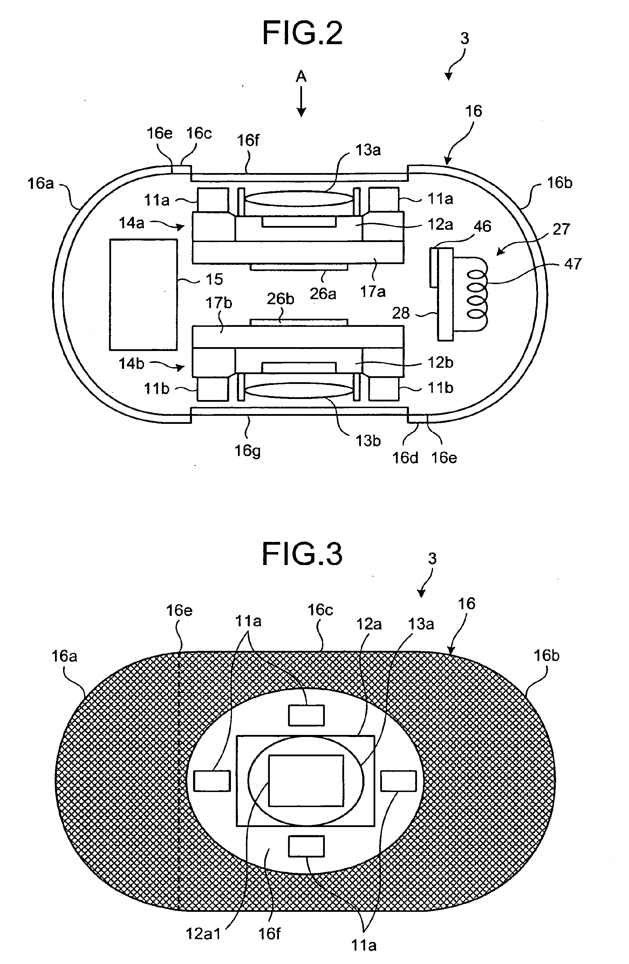

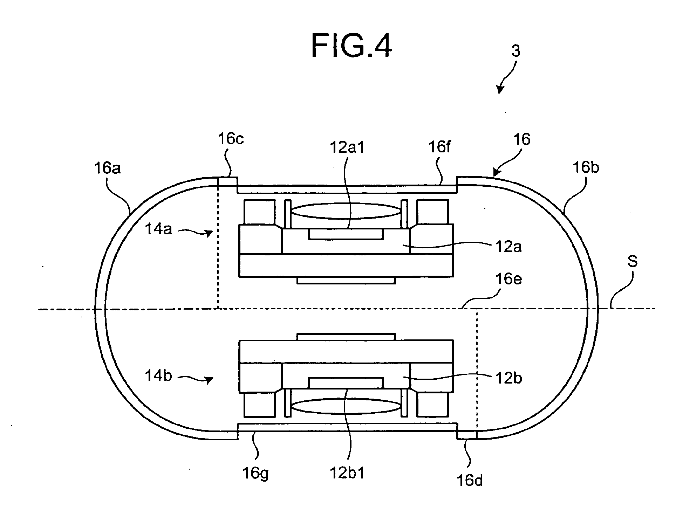

The capsule endoscope 3 will be explained next. FIG. 2 is a side cross-section showing a schematic configuration of the capsule endoscope 3 according to the first embodiment, FIG. 3 is an arrow view showing a view taken from a direction of an arrow A shown in FIG. 2, and FIG. 4 is a side cross-section of a capsule casing of that shown in FIG. 2. A radio unit and a power source unit to be described later are omitted in FIG. 4. As shown in FIGS. 2 and 3, the capsule endoscope 3 is constructed by disposing, for example, LEDs 11a and 11d serving as an illuminating unit that illuminates inside of body cavities of the subject 1, CCDs 12a and 12b serving as an imaging unit that acquires images inside the body cavities, and imaging units 14a and 14b that include imaging lenses 13a and 13b that provide images of an imaging subject to the CCDs 12a and 12b, together with a power source unit 15 that supplies power to these components, in a capsule casing 16 as an exterior case.

The capsule casing 16 is constituted of hemispheric dome-shaped end cover casings 16a and 16b, and semicylindrical body cover casings 16c and 16d that are integrally formed with the above end cover casings 16a and 16b arranged at one end respectively. The end cover casing 16a and the body cover casing 16c, and the end cover casing 16b and the body cover casing 16d are formed to have substantially L-shaped sides, are axisymmetric with respect to a center axis S of a traveling direction of the capsule endoscope 3, and are water-tightly connected with each other along a parting line 16e indicated with a dotted line in the figure with a connecting means such as an adhesive. Specifically, the body cover casings 16c and 16d are connected with each other on side surfaces thereof to form a cylindrical body cover, and end surfaces of the body cover casings 16c and 16d are connected with end surfaces of the end cover casings 16a and 16b. Thus, the exterior case of the capsule endoscope 3 is formed.

Although it is possible to set this parting line 16e so as to bisect the capsule endoscope 3 at the above center axis S, by setting along the dotted line shown in FIG. 4, it is possible to obtain a wide internal space so that components such as the imaging units 14a and 14b and the like that are housed inside the capsule endoscope 3 can easily be put. Furthermore, according to the parting line 16e of the present embodiment, it is possible to enhance mechanical strength of the capsule endoscope 3 when the capsule endoscope 3 is assembled.

Moreover, on portions on a circumferential surface of the body cover casings 16c and 16d, planar portions 16f and 16g each forming a plane with a transparent member having optical transparency are integrally formed with the body cover casings 16c and 16d. The planar portions 16f and 16g are formed to recess from the circumferential surface of the body cover casings 16c and 16d so as to form a concave cross-section (see FIG. 4). The planar portions 16f and 16g are arranged at positions to be opposed with each other when the body cover casings 16c and 16d are connected.

Below the planar portions 16f and 16g inside the body cover casings 16c and 16d of the capsule casing 16, the imaging unit 14a and 14b are arranged respectively, to enable imaging of an imaging subject (inside of body cavities) outside thereof through the above planar portions 16f and 16g. The imaging units 14a and 14b are arranged such that light receiving surfaces 12a1 and 12b1 of the CCDs 12a and 12b are parallel to the planar portions 16f and 16g, thereby enabling imaging in a circumferential direction of the body cover casings 16c and 16d while preventing occurrence of flare and shading (see FIG. 4). The reason of arranging the imaging units 14a and 14b at the positions at which the imaging in the circumferential direction of the body cover casings 16c and 16d is because it is possible to most closely approach the imaging subject inside luminal organs when passing through luminal organs such as esophagus, and the above configuration is adopted to improve image quality of images to be acquired.

Furthermore, the end cover casings 16a and 16b and the body cover casings 16c and 16d are subjected to, for example, a blacking process and the like as shown in FIG. 3 so as to shield light, to prevent occurrence of flare due to reflected light caused because of close approach to the imaging subject. Such light shielding can be made effective also on the planar portions 16f and 16g. In this case, portions except portions above the LEDs 11a and 11b of the illuminating unit and the light receiving surfaces 12a1 and 12b1 of the CCDs 12a and 12b are shielded from light by the black processing and the like.

Moreover, the LEDs 11a and 11b are mounted on illuminating/imaging boards 17a and 17b together with the CCDs 12a and 12b, at four points of left, right, above, and below near an optical axis center of the imaging lenses 13a and 13b. Furthermore, signal processing/control units 26a and 26b to perform processes and to control each component for each of the imaging units 14a and 14b are mounted behind the illuminating/imaging boards 17a and 17b.

In the capsule endoscope 3 of the present embodiment, a button battery 15 as the power source unit is housed inside the end cover casing 16a. For this battery 15, a silver oxide battery, a rechargeable battery, a power generating battery, or the like is used. Moreover, inside the end cover casing 16b, a radio board 28 on which an antenna 47 is mounted is arranged, and behind this radio board 28, a transmitting module 46 as a transmitting unit that transmits image data acquired by the imaging units 14a and 14b by radio communication is mounted. This transmitting module 46 and the antenna 47 constitute a radio unit 27. The signal processing/control units 26a and 26b on the illuminating/imaging boards 17a and 17b and the radio unit 27 on the radio board 28 are electrically connected appropriately with cables and the like, and the battery 15 is also electrically connected appropriately with cables and the like to the signal processing/control units 26a and 26b, the radio unit 27, or the like. The radio unit 27 can be provided independently for each of the imaging units 14a and 14b without being shared by the imaging units 14a and 14b.

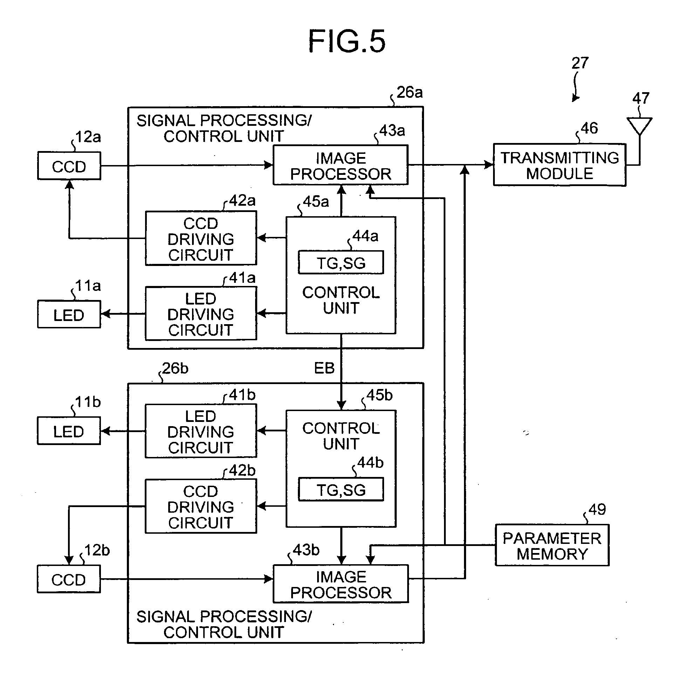

An internal circuit configuration of the capsule endoscope 3 will be explained next with reference to FIG. 5. FIG. 5 is a schematic block diagram showing the internal circuit configuration of the capsule endoscope. First, the signal processing/control unit 26a is to control a pair of the LED 11a and the CCD 12a, and includes an LED driving circuit 41a and a CCD driving circuit 42a corresponding to the LED 11a and the CCD 12a, respectively. Moreover, the signal processing/control unit 26a includes an image processor 43a that performs a predetermined image processing, such as a correlated double sampling process, an amplifying process, an A/D converting process, and a multiplexing process, on an output signal that is output from the CCD 12a. The signal processing/control unit 26a further includes a control unit 45a having a timing generator (TG) and a sync generator (SG) 44a that generate various timing signals and a synchronization signal, and controls an operation of the driving circuits 41a and 42a and the image processor 43a and operation timing thereof based on the timing signal and the synchronization signal generated by the timing generator and the sync generator 44a.

Moreover, the signal processing/control unit 26b is to control a pair of the LED 11b and the CCD 12b, and includes an LED driving circuit 41b and a CCD driving circuit 42b corresponding to the LED 11b and the CCD 12b, respectively. Moreover, the signal processing/control unit 26b includes an image processor 43b that performs a predetermined image processing, such as a correlated double sampling process, an amplifying process, an A/D converting process, and a multiplexing process, on an output signal that is output from the CCD 12b. The signal processing/control unit 26b further includes a control unit 45b having a timing generator (TG) and a sync generator (SG) 44b that generate various timing signals and a synchronization signal, and controls an operation of the driving circuits 41b and 42b and the image processor 43b and operation timing thereof based on the timing signal and the synchronization signal generated by the timing generator and the sync generator 44b.

The control units 45a and 45b have a master-slave relationship such that, for example, the control unit 45a is a master and the control unit 45b is a slave, and the control unit 45b performs a control operation following the control unit 45a in accordance with an enable signal ER from the control unit 45a so as to operate only during this enable signal ER is at a high level. Moreover, the transmitting module 46 and the antenna 47 in the radio unit 27 are arranged on an output path of image data that has passed through the image processors 43a and 43b, and output an RF modulated signal.

Furthermore, the capsule endoscope 3 includes a parameter memory 49 as a common storage unit for the signal processing/control units 26a and 26b. This parameter memory 49 stores parameters unique to each element that are necessary for a signal processing of the image data of each of the CCD 12a and 12b and common parameters that are necessary for a processing of the capsule endoscope 3 so as to be read out by the signal processing/control units 26a and 26b. The parameters are read out at appropriate timing for the image processors 43a and 43b to be used in the multiplexing process with the image data and the like, and can be transmitted to the receiving device 2 through the radio unit 27.

Thus, in the present embodiment, the planar portions 16f and 16g are formed on portions of the circumferential surface of the cylindrical body cover casings 16c and 16d of the capsule casing 16 as an exterior case, and the imaging units 14a and 14b are arranged at the positions at which the imaging of the outside can be performed through the planar portions 16f and 16g. Therefore, the lens effect is prevented and images are improved by eliminating image failures, thereby obtaining appropriate images not affected by the circumferential surface of the exterior case. Thus, the image quality can be improved.

### SECOND EMBODIMENT

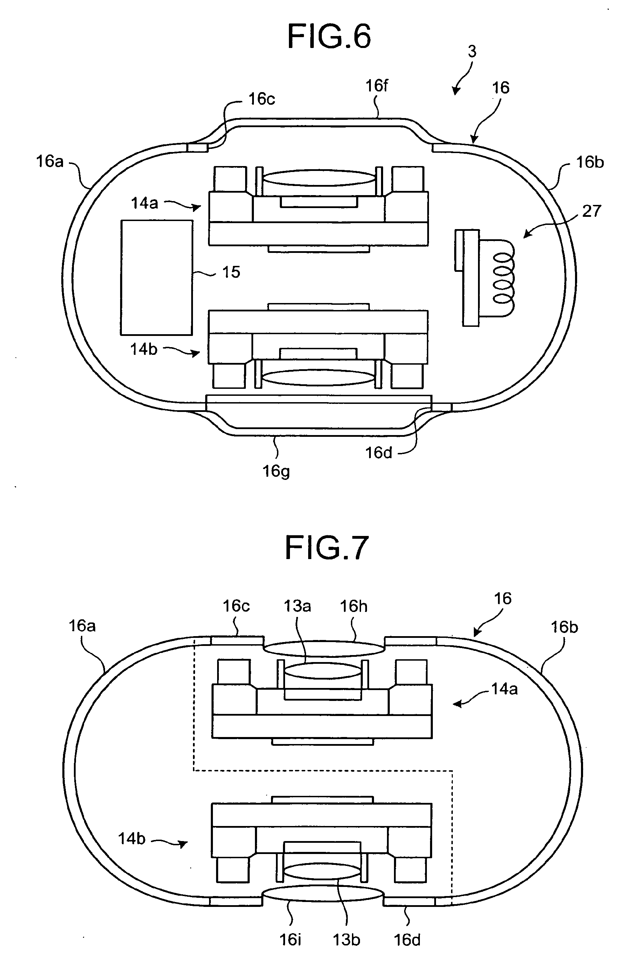

FIG. 6 is a side cross-section showing a schematic configuration of a capsule endoscope according to a second embodiment. In the example shown in FIG. 6, a point different from the capsule endoscope 3 of the first embodiment is that the planar portions 16f and 16g are formed to protrude a little from the circumferential surface of the body cover casings 16c and 16d so as to form a convex cross-section.

Thus, in the capsule endoscope 3 of the second embodiment, the planar portions 16f and 16g protrude from the circumferential surface of the body cover casings 16c and 16d. Therefore, unlike the capsule endoscope 3 of the first embodiment, a field of view is prevented from being narrowed by dust and the like accumulated at the concave planar portions, dust and the like are not accumulated, and a wide field of view is secured.

### THIRD EMBODIMENT

FIG. 7 is a side cross-section showing a schematic configuration of a capsule endoscope according to a third embodiment. The radio unit and the power source unit are omitted in FIG. 7. In the example shown in FIG. 7, points different from the capsule endoscope 3 of the first embodiment are that lens units 16h and 16i constituted of objective lenses having a curvature are formed on portions of the circumferential surface of the body cover casings 16c and 16d instead of the planar portions 16f and 16g, and the imaging units 14a and 14b are arranged below the lens units 16h and 16i respectively to enable the imaging of an imaging subject (inside of body cavities) outside through the above lens units 16h and 16i, and that the end cover casings 16a and 16b and the body cover casings 16c and 16d are subjected to the blacking process and the like to shield light except the lens units 16h and 16i and portions above the LEDs 11a and 11b of the illuminating unit.

Thus, in the present embodiment, the lens units 16h and 16i are formed at portions of the circumferential surface of the body cover casings 16c and 16d, and the imaging units 14a and 14b are arranged at positions at which the imaging of the outside can be performed through the lens units 16h and 16i. Therefore, similarly to the first embodiment, images are improved by eliminating image failures, thereby obtaining appropriate images not affected by the circumferential surface of the exterior case. Thus, the image quality can be improved.

In addition, in the present embodiment, the lens units 16h and 16i constituted of the objective lenses are formed at portions of the circumferential surface of the body cover casings 16c and 16d. Therefore, the imaging lenses 13a and 13b can be removed, thereby enabling to make the imaging units 14a and 14b compact and achieving a space-saving configuration.

While in the above embodiments, a case in which plural imaging units are provided has been explained, the present invention is not limited thereto, and can be configured similarly in a case of a single imaging unit, of course, and effects similar to the above embodiments can be obtained.

### INDUSTRIAL APPLICABILITY

As described above, the body-insertable apparatus according to the present invention is useful for a medical examination device to examine a subject portion while being inserted inside a human body, and is particularly suitable for improving image quality by eliminating image failures to improve images.

## Claims

1. A body-insertable apparatus inserted inside a body of a subject to capture an image inside the body of the subject, comprising:
an exterior case that has at least a cylindrical circumferential surface and closes an inside thereof in a water tight manner;
a planar portion that forms a plane at a portion of the cylindrical circumferential surface; and
an imaging unit that is arranged inside the exterior case at a position at which an imaging of an outside is possible through the planar portion.

2. The body-insertable apparatus according to claim 1, wherein the planar portion is formed so as to dent from the circumferential surface to have a concave cross-section.

3. The body-insertable apparatus according to claim 1, wherein the planar portion is formed so as to protrude from the circumferential surface to have a convex cross-section.

4. The body-insertable apparatus according to claim 1, wherein the exterior case is formed with a first and a second case members having an axisymmetric shape with respect to a center axis of a traveling direction of the body-insertable apparatus.

5. The body-insertable apparatus according to claim 1, wherein the planar portion excluding an imaging region of the imaging unit and the exterior case are formed with a light shielding member.

6. A body-insertable apparatus inserted into a body of a subject to capture an image inside the body of the subject, comprising:
an exterior case that has at least a cylindrical circumferential surface and closes an inside thereof in a water tight manner;
a lens unit that is formed to have a curvature at a portion of the cylindrical circumferential surface; and
an imaging unit that is arranged inside the exterior case at a position at which an imaging of an outside is possible through the lens unit.

7. The body-insertable apparatus according to claim 6, wherein the exterior case excluding the lens unit is formed with a light shielding member.
